# EUROPEAN PATENT SPECIFICATION

(11) **EP 0 496 787 B1**
(45) Date of publication and mention of the grant of the patent: **13.11.1996**
(21) Application number: 90915645.7
(22) Date of filing: 27.09.1990
(51) Int. Cl.: C12N 7/00, A61K 39/12, G01N 33/569

(54) **NOVEL INFECTIOUS BURSAL DISEASE VIRUS**
FÜR DIE INFEKTIÖSE ERKRANKUNG DER BURSA FABRICIUS VERANTWORTLICHER VIRUS
NOUVEAU VIRUS DE LA MALADIE DE LA BOURSE DE FABRICIUS

(30) Priority: 18.10.1989 US 423757
(43) Date of publication of application: 05.08.1992
(73) Proprietor: THE UNIVERSITY OF MARYLAND AT COLLEGE PARK, College Park, Maryland 20742 (US)
(72) Inventor: SNYDER, David, Bowie, MD 20715 (US)
(74) Representative: Signore, Robert
(86) International application number: US9005428
(87) International publication number: WO9105569

(56) References cited:
- WO-A-90/01336
- US-A- 4 530 831
- US-A- 4 824 668
- US-A- 4 956 452
- Journal General Virology, Volume 66, issued December 1985, FAHEY et al. "Antibody to the 32K structural protein of Infections Bursal Disease Virus Neutralizes Viral Infectivity in vitro and confers protection on young chickens" pp. 2693-2702.
- Avian Diseases, Volume 31, issued 1987, CHO et al "An immunoperoxidase Monoclonal antibody stain for Rapid diagnosis of Infectious Bursal Disease" pp. 538-545.

## Description

### Technical Field

This invention relates to the poultry industry, and in particular, infectious bursal disease, a known scourge of this industry. Specifically, a novel virus is identified, and methods of using this virus and information associated therewith are disclosed.

### Background Art

Infectious bursal disease (IBD) has previously been identified as a significant economic drain in the poultry industry. This disease, which strikes chiefly at the chicken industry, is caused by virulent field viruses which cause a highly contagious, immunosupprive disease condition. This condition, of course, exacerbates other infections in the chicken population. The disease is noted for its impact on young chickens, and is characterized by lesions in the lymphoidal follicles of the bursa of Fabricius.

In US-A-4,824,668, it is reported that an attenuated infectious bursal disease virus can be prepared from the virulent strain 1084 variant E and can be used to prepare a vaccine.

In U.S. Patent US-A-5064646 the inventor herein, IBD reported the identification of a novel virus not neutralized by available vaccine and not neutralized by antibodies previously developed as sensitive to, and capable of neutralizing, all known viruses identified as inducing IBD. US-A-5064646 reports the deposition, under Budapest Treaty Conditions, of two viruses, at the Institute Pasteur, under accession numbers i-792 and i-793. This virus, now referred to as GLS, was detected by the use of monoclonal antibodies, particularly those identified as R63 and B69, expressed by hybridomal cell lines deposited under ATCC HB-9437 and HB-9490. These Mab's, identified as neutralizing monoclonal antibodies, comprise a passive vaccine against known strains of viruses inducing IBD and act as a means for detecting the presence of GLS virus, since the positive binding by a nonneutralizing antibody, such as B29, coupled with a negative reaction for R63 and B69 is proof of the GLS IBDV presence.

Thus, recent history in the poultry industry, particularly that along the eastern coast of the United States, reflects an increasingly large number of reports of outbreaks of infectious bursal disease, which are not fully prevented by any of the known vaccines, including those prepared from the monoclonal antibodies discussed above. Due to the severe economic strain placed on the poultry industry by these uncontrolled outbreaks, a significant degree of investigation of the cause of the outbreaks, and the reason for the failure of known vaccines to prevent such outbreaks, has been undertaken. No fault has been detected in the preparation of the vaccines, or their administration. Nonetheless, unchecked outbreaks continue to occur.

This continual outbreak is addressed, in part, by a vaccine developed using the virus addressed in U.S.-A-5064646 This vaccine (GLS vaccine), now being successfully commercially introduced, 5 alone or together with more conventional vaccines, provides protection against the dominant forms of IBDV infections.

New research, using monoclonal antibodies specific to 3 general categories of IBDV, has provided greater understanding of the IBDV.

We report the development of neutralizing Mabs such as 179 and 8, available at the ATCC under deposit numbers HB10158, HB-10174, and neutralizing against all previously known IBDV, and 57, ATCC deposit number HB-10156, neutralizing and specific to the GLS strain and variants. Recently research has identified yet a new IBDV in the field, not controlled by the GLS-vaccine. Monoclonal antibody 179 does not even react in vitro.

Thus, there is yet a new IBDV in the field, against which there is no current active vaccine, and against which no passive vaccine has yet been provided. This new virus, apparently a GLS variant and mutation thereof, given the Mab 57 in vitro binding, is currently uncontrolled. Protection for the poultry industry against this new IBDV is therefore a pressing need.

### Disclosure of the Invention

It has now been discovered that a new virus responsible for infectious bursal disease in poultry in the USA is a newly identified, GLS variant strain with altered recognition sites, such that none of the previously developed group reactive and neutralizing monoclonal antibodies are capable of neutralizing or binding to the virus. However, these monoclonal antibodies do neutralize and react with all known IBD vaccines of the current art. The virus has been isolated in essentially pure form and can be identified by the failure of monoclonal antibody 179 to bind thereto, while another common non-neutralizing antibody as well as standard polyclonal antisera available from the USDA will bind thereto in positive fashion and Mab 57 will bind it in vitro. Thus, the new virus may be recognized by a negative test. If Mab 179 will not bind, but B29, polyclonal antisera, or Mab 57 do react, the new GLS-variant virus, designed DS 326, is present. Since 57 reacts with both GLS and DS 326, positive reaction of this Mab, alone, will not disseminate between the two viruses.

The new virus may be used in killed form as killed vaccines inducing antibodies resistant to the new virus, and may be used in attenuated form or otherwise genetically altered to prepare a live or killed vaccine.

### DETAILED DESCRIPTION OF THE INVENTION

As noted above, the new virus is not bound by any monoclonal antibodies specific thereto. Thus, identification of the presence of the new virus cannot be achieved through normal measures. However, by a combination of negative and positive testing, the presence of the virus and isolation of the virus can be achieved.

In particular, the monoclonal antibody designated 179 which neutralizes all previously identified serotype one IBD virus strains and at least one serotype two strain, gives negative results in an antigen capture-ELISA when reacted with the homogenized bursas drawn from chickens which yielded the new viruses. The same results were observed with Mabs B69 and R63, selective for the D78 virus strain and certain classic and Delaware viruses of an earlier art, once thought to be the prevalent strain in the United States. At the same time, another Mab designated B29, expressed by a hybridomal cell line deposited at the ATCC under accession number MB 9746, pursuant to Budapest Treaty conditions, which does not neutralize the virus, nevertheless binds to it, as well as to all known existing virus vaccines. Additionally, the polyclonal IBDV antisera used as a standard, and available from the USDA's national veterinary services laboratory in Ames, Iowa under designation ADU8701, binds, in the antigen capture ELISA, to the novel virus. Mab 57 will bind to the new virus, in vitro, as well as the GLS virus. Those of skill will identify non-neutralizing antibodies which bind to the virus, and can be directly produced as conventional monoclonal antibodies. The invention is not limited to any given Positive test factor. Since the overall size of the virus, in comparison to any available neutralization site is quite large, there will be a large potential field of such positive test factors and polyclonal antisera.

Thus, the presence of the virus can currently be best determined by negative testing in an antigen capture-ELISA for 179, and positive testing of either Mab 8, B29, 57, the polyclonal antisera or other positive Mab. It should be noted, however, that morphological or symptomatic verification of the presence of an IBD virus, coupled with a failure of the 179 to bind to an antigen sample, is clear evidence of the presence of the virus. Further, a positive reaction with Mab 57 indicates either GLS or DS 326 presence.

### IDENTIFICATION OF THE VIRUS PRESENCE

To originally identify the presence of the new virus, chicken populations from a disaster farm were sampled. Bursas from the chicken populations were homogenized by placing one bursa in one ml of SGPA-EDTA buffer and grinding the mixture with a mortar and pestle until fluid-like consistency was obtained. This material was clarified by low speed centrifugation, and the supernatents were analyzed by an AC-ELISA.

In this assay, 96-well Immulon 1 plates (obtained from Dynatech, of Virginia) were coated with 0. 1 ml of two ug/ml of protein A from Staphlycoccus aureus in a coating buffer. After 18 hours at 4°C, the plates were dumped. 1/10 dilutions of acid supernatents collected from hybridoma cultures secreting the 179, and 57 IBD virus specific Mabs were added in the phosphate buffered saline which contained TWEEN 20 and 2% non-fat dried powdered milk, in alternating fashion. After a 24 hours reaction at 4°C, the plates were tapped dry and blocked for 30 minutes at room temperature. After blocking, the plates were emptied and tapped dry. 0.1 ml of serial dilutions of each sample of the homogenized bursal suspensions were added to the coated plates, and after incubation, the plates were emptied, tapped dry and washed three times for three minutes with PBS-T. Then, each well received 0.1 ml of a biotin labelled 179 Mab conjugate, which was diluted in PBS-T + NFDM. After an hour of incubation, the plates were again emptied and washed. Subsequently, 0.1 ml of a streptavidin-horseradish peroxidase conjugated was added to each well. After one hour of incubation the plates were again emptied and washed. This was followed by the addition of a TMB substrate. After a brief incubation period, the tests were read at 650 nm with the aid of an automated spectrophotometer. Thus, the biotinylated Mab was used to signal for positive reactions between the virus and 179, and 57 wells, while a similar AC-ELISA was performed with a polyclonal anti-IBDV sera was used to signal the B29 catches. Alternatively, biotinylated B29 could be used to the same effect. Further, any form of labeling of Mab or polyclonal antibodies may be used.

All strains showed negative for reactivity with 179, but were highly positive for the B29 Mab, which combines in a non-neutralizing fashion as well as reacting with neutralizing antibody 57.

As 179 is a neutralizing antibody for all previously identified IBD viruses, an assay employing only this as the positive non-neutralizing assay is adequate. The added use of B69 and R63 or 57 gives a higher confidence level, and can be used to further define and separate IBDV strains of the prior art.

### CONFIRMATION OF THE PURITY AND VIRULENCE OF THE VIRUS

Samples from the identified strain, which virus is expressed by the deposit at the Institute Pasteur pursuant to Budapest Treaty conditions under accession number i-910 were pooled, and reacted with the 179 Mab and inoculated into SPF chickens. Five days after inoculation, these chickens, and non-inoculated chickens were necropsied. Those birds inoculated with the collected virus, referred to as DS 326 showed lesions consistent only with infectious bursal disease.

For certainty, antisera from the birds was taken at 11 days past inoculation, and was tested by indirect ELISA and showed serologic conversion to IBDV, but to no other related poultry diseases. Bursal samples from these birds were homogenized and passed a second time in the presence of 179 with identical results. In both passages, on a scale of 0-9, reactivity with the B29 and 57 Mab was at level 9, and reactivity with 179 was at level 0. Thus, a pure preparation of a previously unidentified virus, not related to any known vaccine at the R63 and B69 neutralization sites, prepared from virus or otherwise, was identified. Preparation of additional monoclonal antibodies, protein information, and RNA analysis, is underway. This information will provide the necessary base for the preparation of vaccines based on neutralizing, but non-toxic, recombinant virus-like proteins.

Until such "designed" vaccines becomes available, any of the isolated virus preparations each given the designation DS 326 can be used, in killed form, for the preparation of conventional killed vaccines, which do confer immunity against the new virus. The DS 326 strains may be prepared into a vaccine through common methods, which are not per se at part of this invention among the most prominent of which are heat killing and chemical killing, which preserves the essential form of the vaccine to enable the preparation, by the inoculated bird, of protective NV antibodies while rendering it non-virulent. Alternatively, there are known methods of attenuating viruses, including serial passage, cloning of the virus deleting sequences of nucleic acids and site-directed mutagenesis, which will allow the preparation of a live non-virulent virus vaccine. The vaccines may be prepared by simple incorporation of the selected virus derivative and suspending or mixing it in a carrier. Appropriate dosage values can be determined through routine trial and error techniques, sampling for antibody.

As important as the preparation of the new vaccine is, there is not provided a method by which the presence of the virus can be identified in a given poultry population, by a relatively quick and efficient ELISA assay, which, if reaction to 179 is negative, while the reaction to a polyclonal vaccine, 57 or B29, is positive, then the presence of the virus is confirmed. B29 is expressed by a hybridomal cell line which has been deposited, under Budapest Treaty terms at the ATCC under accession number HB 9746.

## Claims (Claims for the following Contracting State(s): AT, BE, CH, LI, DE, DK, FR, GB, IT, LU, NL, SE)

1. A purified preparation of virus consisting essentially of a virus capable of including infectious bursal disease in poultry, to which monoclonal antibody expressed by cell line HB 10158 (ATCC) will not bind, and to which a non-neutralizing test factor selected from the group consisting of a standard polyclonal antiserum against infectious bursal disease virus and the monoclonal antibody expressed by cell line HB 9746 (ATCC) and comprising infectious bursal disease antibodies will bind.

2. The preparation of claim 1, which virus the monoclonal antibody expressed by HB 10156 (ATCC) will bind in vitro.

3. The preparation of claim 1, wherein said virus is suspended in either a non-immunogenic medium or an immunogenic medium.

4. A vaccine for the prevention of infectious bursal disease in poultry, comprising :
the virus of claim 1 and a pharmacologically acceptable carrier therefore.

5. The vaccine of claim 4, wherein said virus is in a killed form.

6. The vaccine of claim 4, wherein said virus is in a live but attenuated form.

7. A test kit for detecting the presence of a virus according to claim 1 capable of inducing infectious bursal disease in a poultry population, comprising :
a preparation of monoclonal antibody expressed by cell line HE 10158 (ATCC),
a preparation of a non-neutralizing test factor comprising antibodies to infectious bursal disease, and
means for supporting said preparations in contact with a sample of bursal tissue drawn from said population.

8. The kit of claim 7, further comprising a preparation of monoclonal antibody expressed by cell line HB 10156 (ATCC).

9. The kit of claim 7, wherein said test factor comprises antibodies selected from the group consisting of the monoclonal antibody expressed by HB 9746 (ATCC) and the antibodies present in a standard polyclonal antiserum against infectious bursal disease virus.

10. A method for detecting the presence of a virus according to claim 1 capable of inducing infectious bursal disease in a poultry population, which virus is not neutralized by a monoclonal antibody expressed by HB 10158 and will bind with a monoclonal antibody expressed by cell line HB 9746 (ATCC) or with the antibodies present in a standard polyclonal antiserum against infectious bursal disease virus comprising :
contacting samples of bursal tissue separately with the monoclonal antibody expressed by HB 10158 (ATCC) and a positive non-neutralizing factor capable of binding to said virus, said factor comprising antibodies to infectious bursal disease virus, and comparing the degree of binding observed between said samples and said monoclonal antibody and test factor,
wherein a high degree of binding between said sample, and said test factor together with a substantial absence of binding between the monoclonal antibody expressed by HB 10158 (ATCC) and said sample is indicative of the presence of said virus.

11. The method of claim 10, wherein said test factor comprises antibodies selected from the group consisting of monoclonal antibody expressed by cell line HB 9746 (ATCC) and the antibodies present in a standard polyclonal antiserum against infectious bursal disease virus.

12. The method of claim 11, wherein said test factor comprises the monoclonal antibodies of HB 10156 (ATCC).

13. A method for detecting the presence of a virus according to claim 1 capable of inducing infectious bursal disease in a poultry population, which virus is not neutralized by the monoclonal antibody expressed by HE 10158 (ATCC), comprising :
detecting the presence of an infectious bursal disease virus in said population by morphological or pathological inspection, and
contacting samples of bursal tissue drawn from said population with said monoclonal antibody, and observing the degree of binding therebetween,
wherein a substantial absence of binding therebetween indicates the presence of said virus.

14. A substantially pure preparation of the virus expressed by the cell line deposited under the accession number i-910 (CNCM, Institute Pasteur).

## Claims (Claims for the following Contracting State(s): ES)

1. A process for preparing a purified virus which comprises isolating from a chicken population a virus capable of including infectious bursal disease in poultry, to which monoclonal antibody expressed by cell line HB 10158 (ATCC) will not bind, and to which a non-neutralizing test factor selected from the group consisting of a standard polyclonal antiserum against infectious bursal disease virus and the monoclonal antibody expressed by cell line HB 9746 (ATCC) and comprising infectious bursal disease antibodies will bind.

2. The process of claim 1, which virus the monoclonal antibody expressed by HB 10156 (ATCC) will bind in vitro.

3. The process of claim 1, wherein said virus is suspended in either a non-immunogenic medium or an immunogenic medium.

4. A process for preparing a vaccine for the prevention of infectious bursal disease in poultry, comprising :
mixing the virus prepared by the process of claim 1, with a pharmacologically acceptable carrier therefore.

5. The process of claim 4, wherein said virus is in a killed form.

6. The process of claim 4, wherein said virus is in a live but attenuated form.

7. A test kit for detecting the presence of a virus according to claim 1 capable of inducing infectious bursal disease in a poultry population, comprising :
a preparation of monoclonal antibody expressed by cell line HE 10158 (ATCC),
a preparation of a non-neutralizing test factor comprising antibodies to infectious bursal disease, and
means for supporting said preparations in contact with a sample of bursal tissue drawn from said population.

8. The kit of claim 7, further comprising a preparation of monoclonal antibody expressed by cell line HB 10156 (ATCC).

9. The kit of claim 7, wherein said test factor comprises antibodies selected from the group consisting of the monoclonal antibody expressed by HB 9746 (ATCC) and the antibodies present in a standard polyclonal antiserum against infectious bursal disease virus.

10. A method for detecting the presence of a virus according to claim 1 capable of inducing infectious bursal disease in a poultry population, which virus is not neutralized by a monoclonal antibody expressed by HB 10158 (ATCC) and will bind with a monoclonal antibody expressed by cell line HB 9746 (ATCC) or with the antibodies present in a standard polyclonal antiserum against infectious bursal disease virus comprising :
contacting samples of bursal tissue separately with the monoclonal antibody expressed by HB 10158 (ATCC) and a positive non-neutralizing factor capable of binding to said virus, said factor comprising antibodies to infectious bursal disease virus, and comparing the degree of binding observed between said samples and said monoclonal antibody and test factor,
wherein a high degree of binding between said sample, and said test factor together with a substantial absence of binding between the monoclonal antibody expressed by HB 10158 (ATCC) and said sample is indicative of the presence of said virus.

11. The method of claim 10, wherein said test factor comprises antibodies selected from the group consisting of monoclonal antibody expressed by cell line HB 9746 (ATCC) and the antibodies present in a standard polyclonal antiserum against infectious bursal disease virus.

12. The method of claim 11, wherein said test factor comprises the monoclonal antibodies of HB 10156 (ATCC).

13. A method for detecting the presence of a virus according to claim 1 capable of inducing infectious bursal disease in a poultry population, which virus is not neutralized by the monoclonal antibody expressed by HB 10158 (ATCC), comprising :
detecting the presence of an infectious bursal disease virus in said population by morphological or pathological inspection, and
contacting samples of bursal tissue drawn from said population with said monoclonal antibody, and observing the degree of binding therebetween,
wherein a substantial absence of binding therebetween indicates the presence of said virus.

14. A process for preparing a substantially pure virus which comprises expressing said virus by the cell line deposited under the accession number i-910, (CNCM, Institute Pasteur)

## Patentansprüche (Patentansprüche für folgende(n) Vertragsstaat(en): AT, BE, CH, LI, DE, DK, FR, GB, IT, LU, NL, SE)

1. Gereingte Viruspräparation, im wesentlichen bestehend aus einem Virus, welches die infektiöse Bursa-Krankheit bei Geflügel hervorrufen kann, an welches der durch die Zelllinie HB 10158 (ATCC) exprimierte monoklonale Antikörper nicht bindet und an welches ein nicht-neutralisierender Testfaktor, ausgewählt aus der Gruppe, bestehend aus einem polyklonalen Standard-Antiserum gegen infektiöses Bursa-Krankheits-Virus und dem monoklonalen Antikörper, der durch die Zellinie HB 9746 (ATCC) exprimiert wird und für infektiöse Bursa-Krankheit spezifische Antikörper umfaßt, bindet.

2. Präparation nach Anspruch 1, worin das Virus den von HB 10156 (ATCC) exprimierten monoklonalen Antikörper in vitro bindet.

3. Präparation nach Anspruch 1, worin das Virus entweder in einem nicht-immunogenen Medium oder in einem immunogenen Medium suspendiert ist.

4. Impfstoff für die Prävention der infektiösen Bursa-Krankheit bei Geflügel, umfassend
das Virus nach Anspruch 1 und einen pharmakologisch annehmbaren Träger.

5. Impfstoff nach Anspruch 4, worin das Virus in einer abgetöteten Form vorliegt.

6. Impfstoff nach Anspruch 4, worin das Virus in einer lebenden, jedoch attenuierten Form vorliegt.

7. Testkit zum Nachweisen der Gegenwart eines Virus nach Anspruch 1, welches infektiöse Bursa-Krankheit in einer Geflügelpopulation hervorrufen kann, umfassend
eine Präparation des durch die Zellinie HB 10158 (ATCC) exprimierten monoklonalen Antikörpers,
eine Präparation eines nicht-neutralisierenden Testfaktors, umfassend Antikörper gegen infektiöse Bursa-Krankheit, und
Mittel zum Fördern des Kontakts der Präparationen mit einer Probe von Bursa-Gewebe, das von der Population genommen wurde.

8. Kit nach Anspruch 7, weiterhin umfassend eine Präparation eines durch die Zellinie HB 10156 (ATCC) exprimierten monoklonalen Antikörpers.

9. Kit nach Anspruch 7, worin der Testfaktor Antikörper, ausgewählt aus der Gruppe, bestehend aus den durch HB 9746 (ATCC) exprimierten monoklonalen Antikörper und Antikörpern, die in einem polyklonalen Standard-Antiserum gegen infektiöses Bursa-Krankheits-Virus vorhanden sind, umfaßt.

10. Verfahren zum Nachweisen der Gegenwart eines Virus nach Anspruch 1, welches infektiöse Bursa-Krankheit in einer Geflügelpopulation hervorrufen kann, wobei das Virus nicht durch einen durch HB 10158 (ATCC) exprimierten monoklonalen Antikörper neutralisiert wird und an einen durch die Zellinie HB 9746 (ATCC) exprimierten monoklonalen Antikörper oder an Antikörper, die in einem polyklonalen Standard-Antiserum gegen infektiöses Bursa-Krankheits-Virus vorhanden sind, bindet, umfassend:
getrenntes Inkontaktbringen der Proben von Bursa-Gewebe mit dem durch HB 10158 (ATCC) exprimierten monoklonalen Antikörper und einem positiven, nicht-neutralisierenden Faktor, der an das Virus binden kann, wobei der Faktor Antikörper gegen infektiöses Bursa-Krankheits-Virus umfaßt, und Vergleichen des Bindungsgrades, der zwischen den Proben und dem monoklonalen Antikörper und Testfaktor beobachtet wird, worin ein hoher Bindungsgrad zwischen der Probe und dem Testfaktor zusammen mit einer wesentlichen Abwesenheit der Bindung zwischen dem durch HB 10158 (ATCC) exprimierten monoklonalen Antikörper und der Probe ein Anzeichen für die Gegenwart des Virus ist.

11. Verfahren nach Anspruch 10, worin der Testfaktor Antikörper umfaßt, die ausgewählt sind aus der Gruppe, bestehend aus dem durch die Zellinie HB 9746 (ATCC) exprimierten monoklonalen Antikörper und Antikörpern, die in einem polyklonalen Standard-Antiserum gegen infektiöses Bursa-Krankheits-Virus vorhanden sind.

12. Verfahren nach Anspruch 11, worin der Testfaktor monoklonale Antikörper von HB 10156 (ATCC) umfaßt.

13. Verfahren zum Nachweisen der Gegenwart eines Virus nach Anspruch 1, welches infektiöse Bursa-Krankheit in einer Geflügelpopulation hervorrufen kann, wobei das Virus nicht durch den durch HB 10158 (ATCC) exprimierten monoklonalen Antikörper neutralisiert wird, umfassend:
Nachweisen der Gegenwart eines infektiösen Bursa-Krankheits-Virus in der Population durch morphologische oder pathologische Überprüfung und
Inkontaktbringen der Proben von Bursa-Gewebe, welches von der Population genommen wurde, mit dem monoklonalen Antikörper und Beobachten des Bindungsgrades,
worin eine wesentliche Abwesenheit der Bindung zwischen diesen die Gegenwart des Virus anzeigt.

14. Im wesentlichen reine Präparation des Virus, welches durch die Zellinie, die unter der Hinterlegungsnummer i-910 (CNCM, Pasteur-Institut) hinterlegt ist, exprimiert wird.

## Patentansprüche (Patentansprüche für folgende(n) Vertragsstaat(en): ES)

1. Verfahren zum Herstellen eines gereinigten Virus, welches das Isolieren eines Virus, welches infektiöse Bursa-Krankheit in Geflügel hervorrufen kann, an welches der durch die Zellinie HB 10158 (ATCC) exprimierte monoklonale Antikörper nicht bindet und an welches ein nicht-neutralisierender Testfaktor, ausgewählt aus der Gruppe, bestehend aus einem polyklonalen Standard-Antiserum gegen infektiöses Bursa-Krankheits-Virus und dem monoklonalen Antikörper, der durch die Zellinie HB 9746 (ATCC) exprimiert wird und für infektiöse Bursa-Krankheit spezifische Antikörper umfaßt, bindet, aus einer Hühnerpopulation umfaßt.

2. Verfahren nach Anspruch 1, worin das Virus den durch HB 10156 (ATCC) exprimierten monoklonalen Antikörper in vitro bindet.

3. Verfahren nach Anspruch 1, worin das Virus in entweder einem nicht-immunogenen Medium oder einem immunogenen Medium suspendiert ist.

4. Verfahren zum Herstellen eines Impfstoffs für die Prävention von infektiöser Bursa-Krankheit in Geflügel, umfassend:
Mischen des durch das Verfahren nach Anspruch 1 hergestellten Virus mit einem pharmakologisch annehmbaren Träger.

5. Verfahren nach Anspruch 4, worin das Virus in einer abgetöteten Form vorliegt.

6. Verfahren nach Anspruch 4, worin das Virus in einer lebenden, jedoch attendierten Form vorliegt.

7. Testkit zum Nachweisen der Gegenwart eines Virus nach Anspruch 1, welches infektiöse Bursa-Krankheit in einer Geflügelpopulation hervorrufen kann, umfassend:
eine Präparation des durch die Zellinie HB 10158 (ATCC) exprimierten monoklonalen Antikörpers,
eine Präparation eines nicht-neutralisierenden Testfaktors, umfassend Antikörper gegen infektiöse Bursa-Krankheit, und
Mittel zum Fördern des Kontakts der Präparationen mit einer Probe von Bursa-Gewebe, das von der Population genommen wurde.

8. Kit nach Anspruch 7, weiterhin umfassend eine Präparation eines durch die Zellinie HB 10156 (ATCC) exprimierten monoklonalen Antikörpers.

9. Kit nach Anspruch 7, worin der Testfaktor Antikörper, ausgewählt aus der Gruppe, bestehend aus den durch HB 9746 (ATCC) exprimierten monoklonalen Antikörper und Antikörpern, die in einem polyklonalen Standard-Antiserum gegen infektiöses Bursa-Krankheits-Virus vorhanden sind, umfaßt.

10. Verfahren zum Nachweisen der Gegenwart eines Virus nach Anspruch 1, welches infektiöse Bursa-Krankheit in einer Geflügelpopulation hervorrufen kann, wobei das Virus nicht durch einen durch HB 10158 (ATCC) exprimierten monoklonalen Antikörper neutralisiert wird und an einen durch die Zellinie HB 9746 (ATCC) exprimierten monoklonalen Antikörper oder an Antikörper, die in einem polyklonalen Standard-Antiserum gegen infektiöses Bursa-Krankheits-Virus vorhanden sind, bindet, umfassend:
getrenntes Inkontaktbringen der Proben von Bursa-Gewebe mit dem durch HB 10158 (ATCC) exprimierten monoklonalen Antikörper und einem positiven, nicht-neutralisierenden Faktor, der an das Virus binden kann, wobei der Faktor Antikörper gegen infektiöses Bursa-Krankheits-Virus umfaßt, und Vergleichen des Bindungsgrades, der zwischen den Proben und dem monoklonalen Antikörper und Testfaktor beobachtet wird, worin ein hoher Bindungsgrad zwischen der Probe und dem Testfaktor zusammen mit einer wesentlichen Abwesenheit der Bindung zwischen dem durch HB 10158 (ATCC) exprimierten monoklonalen Antikörper und der Probe ein Anzeichen für die Gegenwart des Virus ist.

11. Verfahren nach Anspruch 10, worin der Testfaktor Antikörper umfaßt, die ausgewählt sind aus der Gruppe, bestehend aus dem durch die Zellinie HB 9746 (ATCC) exprimierten monoklonalen Antikörper und Antikörpern, die in einem polyklonalen Standard-Antiserum gegen infektiöses Bursa-Krankheits-Virus vorhanden sind.

12. Verfahren nach Anspruch 11, worin der Testfaktor monoklonale Antikörper von HB 10156 (ATCC) umfaßt.

13. Verfahren zum Nachweisen der Gegenwart eines Virus nach Anspruch 1, welches infektiöse Bursa-Krankheit in einer Geflügelpopulation hervorrufen kann, wobei das Virus nicht durch den durch HB 10158 (ATCC) exprimierten monoklonalen Antikörper neutralisiert wird, umfassend:
Nachweisen der Gegenwart eines infektiösen Bursa-Krankheits-Virus in der Population durch morphologische oder pathologische Überprüfung und
Inkontaktbringen der Proben von Bursa-Gewebe, welches von der Population genommen wurde, mit dem monoklonalen Antikörper und Beobachten des Bindungsgrades,
worin eine wesentliche Abwesenheit der Bindung zwischen diesen die Gegenwart des Virus anzeigt.

14. Verfahren zum Herstellen eines reinen Virus, welches das Exprimieren des Virus durch die Zellinie, welche unter der Hinterlegungsnummer i-910 (CNCM, Pasteur-Institut) hinterlegt ist, umfaßt.

## Revendications (Revendications pour l'(les) Etat(s) contractant(s) suivant(s): AT, BE, CH, LI, DE, DK, FR, GB, IT, LU, NL, SE)

1. Composition purifiée à base d'un virus consistant essentiellement en un virus capable de provoquer une bursite infectieuse chez la volaille, auquel un anticorps monoclonal, exprimé par la lignée de cellules ATCC HB 10158, ne se lie pas, et auquel se lie un facteur d'essai non neutralisant choisi parmi un antisérum polyclonal standard dirigé contre un virus de bursite infectieuse et l'anticorps monoclonal exprimé par la lignée de cellules ATCC HB 9746, et comprenant des anticorps contre la bursite infectieuse.

2. Composition selon la revendication 1, l'anticorps monoclonal exprimé par la lignée ATCC HB 10156 se liant au virus *in vitro.*

3. Composition selon la revendication 1, dans laquelle ledit virus est en suspension dans un milieu non immunogène ou un milieu immunogène.

4. Vaccin pour la prévention de la bursite infectieuse chez la volaille, comprenant : le virus de la revendication 1 et un véhicule pharmaceutiquement acceptable pour celui-ci.

5. Vaccin selon la revendication 4, dans lequel ledit virus est sous forme tuée.

6. Vaccin selon la revendication 4, dans lequel ledit virus est sous forme vivante mais atténuée.

7. Nécessaire d'essai pour détecter la présence d'un virus selon la revendication 1, capable de provoquer une bursite infectieuse dans une population de volailles, comprenant :
une préparation d'anticorps monoclonal exprimée par la lignée de cellules ATCC HE 10158,
une préparation de facteur d'essai non neutralisant comprenant des anticorps dirigés contre la bursite infectieuse, et
des moyens pour supporter ces préparations en contact avec un échantillon de tissu de bourse prélevé à partir de ladite population.

8. Nécessaire selon la revendication 7, comprenant en outre une préparation d'un anticorps monoclonal exprimé par la lignée de cellules ATCC HB 10156.

9. Nécessaire selon la revendication 7, dans lequel le facteur d'essai comprend des anticorps choisis du groupe consistant en l'anticorps monoclonal exprimé par la lignée ATCC HB 9746 et les anticorps présents dans un antisérum polyclonal standard dirigé contre un virus de bursite infectieuse.

10. Procédé de détection de la présence d'un virus selon la revendication 1, capable de provoquer une bursite infectieuse dans une population de volailles, ce virus n'est pas neutralisé par un anticorps monoclonal exprimé par la lignée HB 10158, et il se lie avec un anticorps monoclonal exprimé par la lignée de cellules ATCC HB 9746 ou avec les anticorps présents dans un antisérum polyclonal standard dirigé contre un virus de bursite infectieuse, selon lequel :
on met séparément en contact des échantillons de tissu de bourse avec l'anticorps monoclonal exprimé par la lignée ATCC HB 10158 et un facteur positif non neutralisant capable de se lier audit virus, ce facteur comprenant des anticorps dirigés contre un virus de bursite infectieuse, et on compare le degré de liaison observé entre les échantillons et l'anticorps monoclonal et le facteur d'essai, dans lequel un degré élevé de liaison entre l'échantillon, et ledit facteur d'essai en association avec une absence notable de liaison entre l'anticorps monoclonal exprimé par la lignée ATCC HB 10158 et l'échantillon, est indicatif de la présence dudit virus.

11. Procédé selon la revendication 10, dans lequel ledit facteur d'essai comprend des anticorps choisis dans le groupe consistant en un anticorps monoclonal exprimé par la lignée de cellules ATCC HB 9746 et les anticorps présents dans un antisérum polyclonal standard dirigé contre un virus de bursite infectieuse.

12. Procédé selon la revendication 10, dans lequel ledit facteur d'essai comprend les anticorps monoclonaux de la lignée ATCC HB 10156.

13. Procédé de détection de la présence d'un virus selon la revendication 1, capable de provoquer une bursite infectieuse dans une population de volailles, ce virus n'est pas neutralisé par l'anticorps monoclonal exprimé par la lignée ATCC HB 10158, selon lequel :
on détecte la présence d'un virus de bursite infectieuse dans ladite population, par examen morphologique ou pathologique, et
on met en contact des échantillons de tissu de bourse prélevés à partir de ladite population, avec ledit anticorps monoclonal, et on observe le degré de liaison entre ceux-ci,
dans lequel une absence notable de liaison entre ceux-ci indique la présence dudit virus.

14. Préparation substantiellement pure du virus exprimé par la lignée de cellules déposée sous le numéro d'enregistrement i-910 (CNCM, Institut Pasteur).

## Revendications (Revendications pour l'(les) Etat(s) contractant(s) suivant(s): ES)

1. Procédé de préparation d'un virus purifié, selon lequel on isole à partir d'une population de poulets, un virus capable de provoquer une bursite infectieuse chez la volaille, auquel ne se lie pas un anticorps monoclonal exprimé par la lignée de cellules ATCC HB 10158, et auquel se lie un facteur d'essai non neutralisant choisi parmi un antisérum polyclonal standard dirigé contre un virus de bursite infectieuse et l'anticorps monoclonal exprimé par la lignée de cellules ATCC HB 9746, et comprenant des anticorps contre la bursite infectieuse.

2. Procédé selon la revendication 1, l'anticorps monoclonal exprimé par la lignée ATCC HB 10156 se liant au virus *in vitro.*

3. Procédé selon la revendication 1, dans lequel ledit virus est en suspension dans un milieu non immunogène ou un milieu immunogène.

4. Procédé de préparation d'un vaccin pour la prévention de la bursite infectieuse chez la volaille, comprenant :
le mélange du virus préparé selon le procédé de la revendication 1 avec un véhicule pharmaceutiquement acceptable pour celui-ci.

5. Procédé selon la revendication 4, dans lequel ledit virus est sous forme tuée.

6. Procédé selon la revendication 4, dans lequel ledit virus est sous forme vivante mais atténuée.

7. Nécessaire d'essai pour détecter la présence d'un virus selon la revendication 1, capable de provoquer une bursite infectieuse dans une population de volailles, comprenant :
une préparation d'anticorps monoclonal exprimée par la lignée de cellules ATCC HE 10158,
une composition à base de facteur d'essai non neutralisant comprenant des anticorps dirigés contre la bursite infectieuse, et
des moyens pour supporter ces compositions en contact avec un échantillon de tissu de bourse prélevé à partir de ladite population.

8. Nécessaire selon la revendication 7, comprenant en outre une préparation d'un anticorps monoclonal exprimé par la ligne de cellules ATCC HB 10156.

9. Nécessaire selon la revendication 7, dans lequel ledit facteur d'essai comprend des anticorps choisis du groupe consistant en l'anticorps monoclonal exprimé par la lignée ATCC HB 9746, et les anticorps présents dans un antisérum polyclonal standard dirigé contre un virus de bursite infectieuse.

10. Procédé de détection de la présence d'un virus selon la revendication 1, capable de provoquer une bursite infectieuse dans une population de volailles, ce virus n'est pas neutralisé par un anticorps monoclonal exprimé par la lignée ATCC HB 10158, et se liant à un anticorps monoclonal exprimé par la lignée de cellules ATCC HB 9746 ou avec les anticorps présents dans un antisérum polyclonal standard dirigé contre un virus de bursite infectieuse, selon lequel :
on met séparément en contact des échantillons de tissu de bourse avec l'anticorps monoclonal exprimé par la lignée ATCC HB 10158 et un facteur positif non neutralisant capable de se lier audit virus, ledit facteur comprenant des anticorps dirigés contre un virus de bursite infectieuse, et on compare le degré de liaison observé entre lesdits échantillons et ledit anticorps monoclonal et le facteur d'essai, dans lequel un degré élevé de liaison entre l'échantillon, et ledit facteur d'essai, en association avec une absence notable de liaison entre l'anticorps monoclonal exprimé par la lignée ATCC HB 10158 et l'échantillon, est indicatif de la présence dudit virus.

11. Procédé selon la revendication 10, dans lequel ledit facteur d'essai comprend des anticorps choisis dans le groupe consistant en un anticorps monoclonal exprimé par la lignée de cellules ATCC HB 9746 et les anticorps présents dans un antisérum polyclonal standard dirigé contre un virus de bursite infectieuse.

12. Procédé selon la revendication 10, dans lequel ledit facteur d'essai comprend les anticorps monoclonaux de la lignée ATCC HB 10156.

13. Procédé de détection de la présence d'un virus selon la revendication 1, capable de provoquer une bursite infectieuse dans une population de volailles, ce virus n'étant pas neutralisé par l'anticorps monoclonal exprimé par la lignée ATCC HB 10158, selon lequel :
on détecte la présence d'un virus de bursite infectieuse dans ladite population, par examen morphologique ou pathologique, et
on met en contact des échantillons de tissu de bourse prélevés à partir de ladite population, avec ledit anticorps monoclonal, et on observe le degré de liaison entre ceux-ci,
dans lequel une absence notable de liaison entre ceux-ci indique la présence dudit virus.

14. Procédé de préparation d'un virus substantiellement pur, comprenant l'expression de ce virus par la lignée de cellules déposée sous le numéro d'enregistrement i-910 (CNCM, Institut Pasteur).
